# EUROPEAN PATENT APPLICATION

(11) **EP 0 680 961 A1**
(43) Date of publication of application: **08.11.1995**
(21) Application number: 95400844.7
(22) Date of filing: 13.04.1995
(51) Int. Cl.: C07D 401/06, C07D 403/06

(54) **Improved, large-scale process for azapirone synthesis**

(30) Priority: 05.05.1994 US 238551
(71) Applicant: BRISTOL-MYERS SQUIBB COMPANY, Princeton, NJ 08543-4000 (US)
(72) Inventor: Melton, Jack, Syracuse, New York 13207 (US)
(74) Representative: Durand, Yves Armand Louis

(57) **Abstract**

An improved, large-scale process for preparation of certain useful azapirones, e.g., buspirone, gepirone, tandospirone, etc., comprises the anhydrous reaction of a spiroquaternary piperazinium salt with an appropriate preferred imide anion to form the azapirone with heating. This process is designed for large scale production and provides advantages in greater safety, efficiency, and environmental impact in addition to yielding a larger, cleaner amount of azapirone product under mild reaction conditions requiring only about a third of the previous reaction time.

## Description

This invention describes an improved, large-scale process for the synthesis of certain useful azapirone compounds. "Azapirone" is a term that has been used to describe a structural class of psychotropic compounds that demonstrate similar pharmacology relating to interaction with monoaminergic pathways in particular brain regions.

The azapirones amenable to the new process of this invention can be shown by some representative illustrations of certain azapirorie drug agents having structural formula (I).

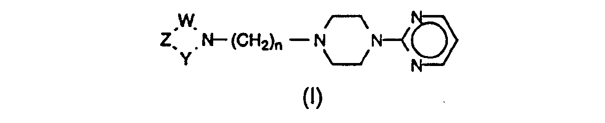

In formula I, W and Y can independently be carbonyl or sulfonyl and n is the integer 4 or 5. Z is selected inter alia from 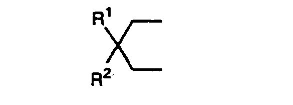 in which R¹ and R² are selected from lower alkyl or are taken together as a butyl or pentyl bridge;

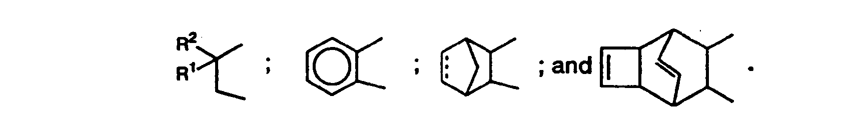

Perhaps the best known representative of the azapirone class of psychotropic agents is buspirone (1), originally disclosed in U.S. 3,71 7,634.

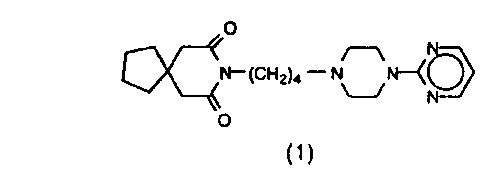

Some other well known members are:
gepirone, where 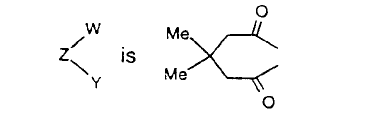
(U.S. 4,423,049); ipsapirone, where 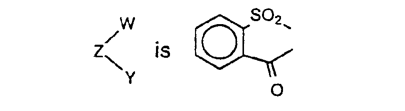
(U.S. 4,818,756); tandospirone, where 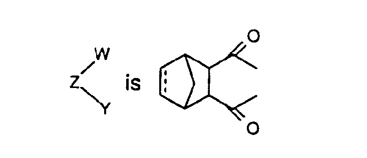
(U.S. 4,507,303); and WY-47,846, where 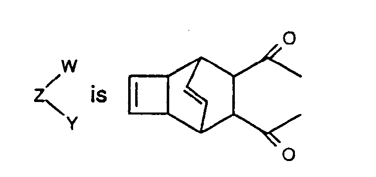
(U.S. 4,892,943).

The dotted and solid lines in the tandospirone-type structure can be taken as either a single or double carbon-carbon covalent bond.

While a number of synthetic processes have been disclosed for the synthesis of these azapirones, a method of choice, currently used for large scale preparation of buspirone and gepirone, was disclosed by Sims in U.S. 4,351,939. The Sims method involves the reaction of an appropriately-substituted glutarimide (3) with a novel spiroquatemary ammonium halide (4) to yield buspirone or gepirone or 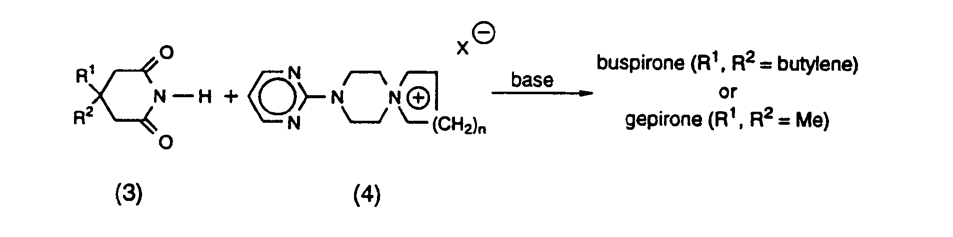 close analogs. The halide, X, is preferably bromide. The reaction is carried out in a hot inert reaction medium in the presence of an acid scavenging base. In practice, the reaction process involves a multiphasic reaction of (3) and (4) in refluxing xylene with an excess of solid potassium carbonate.

For large-scale production, this prior art synthesis suffers from several processing disadvantages, including:
· high temperature processing in toxic solvents, e.g., refluxing xylene;
· a multiphasic reaction mixture requiring highly efficient stirring and as the scale increases, this factor becomes increasingly important;
• the presence of large amounts of inorganic by-products which complicate reaction workup and product isolation;
• long reaction time, e.g., 24 hours; and
• lower and more erratic yields of product resulting from the generation of water as a by-product. The efficient removal of the water is a problem, particularly in these large-scale processes.

Compounds of formula (2) 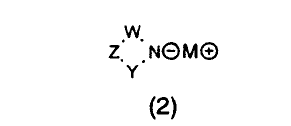 such as imidate anions of structure (2a), 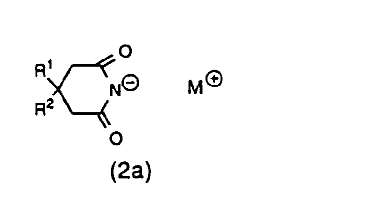 wherein MIB represents an alkali or alkaline earth metal, can be reacted with a pyrimidinylpiperazinyl derivative of formula (5), 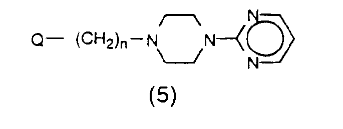 wherein Q is a nucleofuge, i.e. a leaving group of the type commonly utilized in synthetic organic chemistry; by heating in an inert solvent under standard conditions such as those described for the alkylation step of the Gabriel synthesis; cf: Gibson and Bradshaw, Angew. Chem. Int. Ed., 7/919,930, (1968).

The reaction of certain anions, e.g., (2) with intermediates of formula (5), has been previously disclosed. This method has been reported, for example, for the preparation of buspirone (U.S. 3,717,634) and ipsapirone (U.S. 4,818,756). In general, this method has not been used on a large scale, particularly with imides, due to the additional processing requirements necessitated by the generation and handling of a reactive metal salt of the imidate component. As mentioned, the Sims process (involving the reaction of (3) and (4)-type compounds) is the current method of choice for large-scale synthesis of these azapirones.

These prior art processes differ then from the novel improved process which utilizes a pre-formed potassium salt of the imidate-type starting material (2) which is reacted with a spiroquatemary salt (4), instead of a formula (5) compound, to provide azapirone product. The several advantages realized by the adoption of this novel process for large-scale azapirone production, while reflecting the shortcomings of previous processes, are not suggested nor made obvious by the prior art processes.

### Summary of the Invention

The invention concerns a new, improved, large-scale process for the production of certain useful azapirone psychotropic agents such as buspirone, gepirone, ipsapirone, tandospirone, and other structural analogs. The process employs a pre-formed anionic intermediate and a spiroquaternary piperazinium salt and gives reliably higher and cleaneryields of azapirone product under mild anhydrous reaction conditions requiring much shorter reaction and processing times and the avoidance of environmentally objectionable solvents. Reaction and processing conditions are also simplified, thereby further contributing to the production economies realized by utilization of the instant improved process.

### Detailed Description of the Invention

The new, improved large-scale process is illustrated in the flowchart of Scheme I. Illustrative examples are set forth infra as Specific Embodiments.

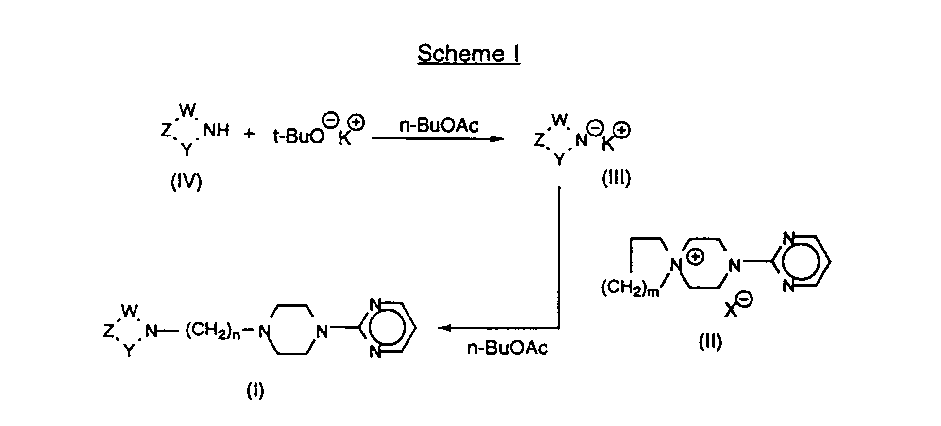

In Scheme I, W and Yare independently selected from carbonyl and sulfonyl with carbonyl being preferred. Z can be a variety of moieties such as 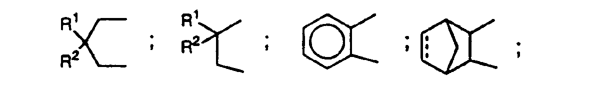 and

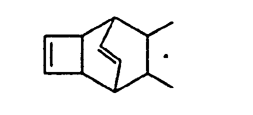

The dotted plus solid line denotes either a single or a double carbon-carbon covalent bond. The symbol m represents the integers 1 and 2, and n represents the integers 4 and 5. R¹ and R² are independently selected from lower alkyl or R¹ and R² can be taken together as a butyl or a pentyl bridge. Lower alkyl is meant to be inclusive of C₁₋₄alkyl groups. The symbol X is commonly understood to be a nucleofuge, that is, a leaving group commonly used in organic synthesis with chloride, bromide and iodide being preferred in the instant process and with bromide being the most preferred.

In the instant process, preferred embodiments involve W and Y being carbonyl and Z being 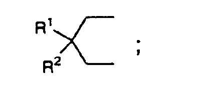 m is 2 and n is 4.

The first step of the Scheme I process involves the preparation of a metallic salt of (IV) with the preferred embodiment being shown which is the potassium salt (III) by treating the starting material (IV) with potassium t-butoxide in a suitable non-protic solvent such as n-butyl acetate or toluene. It should be understood that there are other processes yielding a metallic anion analogous to the potassium salt (III). While these can be considered equivalents, the potassium salt is the preferred species, being surprisingly stable on exposure to air. For use in the improved process of the present invention, the overall yield and quality of the azapirone product are enhanced by the use of pre-formed, relatively pure potassium salt (III).

The key step of the instant process is the reaction of the potassium salt (III) with the spiroquatemary piperazinium salt (II) under mild anhydrous conditions to give azapirone product which is more pure and formed in higher yield than in the prior art processes. By reacting (II) and (III) in the improved process, reaction times are reduced by about two-thirds and lower temperatures can be employed, e.g., reaction temperatures of about 125° and below as compared to about 140° (xylene reflux) as previously required. The lowered temperature requirement permits the selection of more economical and environmentally acceptable solvents--a major consideration for large-scale processes.

The reaction of (II) and (III) also circumvents the use of the acid-scavenging base, a role filled by anhydrous K₂CO₃ in previous large-scale processing. The incorporation of the solid phase carbonate in the final reaction step of the prior art processes resulted in complications due not only to the difficulty of maintaining efficient mixing of the biphasic reaction mixture for 24 hours at the temperature of refluxing xylene, but also due to the large amount of inorganic byproduct that required removal during the reaction workup.

In work leading to the present novel process, it was discovered that the presence of moisture in the reaction media during the reaction of the imide and the spiroquatemary salt led to the erratic lowering of product yield in the process. It was further discovered that even if the media were anhydrous at the start of the reaction, water could be generated as a result of the presence of the alkali carbonate acid scavenger. The source of the water is from the decomposition of bicarbonate ion during the lengthy, hot reaction process. Bicarbonate is formed, for example, by the reaction between K₂CO₃ and IV and between the liberated hydroxide ion, resulting from the thermal breakdown of bicarbonate with IV, with potassium carbonate, e.g.,

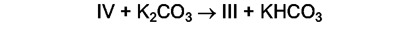

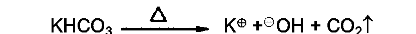

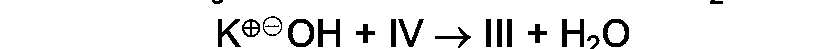

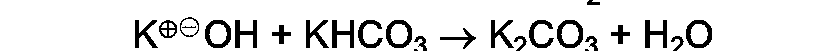

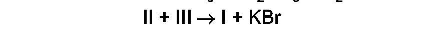

Switching to the improved, large-scale process, which does not suffer from water production under its reaction conditions, a yield increase from about 85% for the prior process to about 93-96% for the new process results. The 85% yield figure given for the prior art process is the average yield recorded over many runs. In actual practice, the yields were erratic. The new, improved process provides yields consistently in the 93-96% range.

Other advantages found with the new, improved process in addition to a consistent higher yield are:
· Milder reaction conditions--the reaction temperature is about 125° compared with temperature greater than about 140° for the previous process.
· More convenient solvents can be employed for the reaction media as a result of the improved process--the previous solvent, xylene, not only has significant negative environmental impact in its handling and disposal but also possessed a tendency to form emulsions during reaction workup, thereby adding time and labor expense to the process.
· A much shorter reaction time--the reaction is complete within 8 hours under conditions of the new process, whereas 24 hours were required previously. This represents a two-thirds reduction in time.
· Elimination of the acid-scavenging base, e.g., solid potassium carbonate--processing requirements for efficient stirring of a biphasic reaction mixture and removal of a large amount of inorganic reaction by-products are eased considerably.

The simplification and shortening of the reaction and workup phases of the process contribute to the economic efficiency of the improved process. The production of cleaner product in the improved process eliminates some purification labor previously required. In the reaction phase, the coupling of an anionic intermediate (III) with a spiroquatemary piperidinium salt (II) conveniently gives the appropriate azapirone drug products under mild conditions and in high yield and purity. By-products formed in prior art high temperature processes are avoided and product isolation is facilitated. With elimination of certain process features and operations required in prior art processes, the novel improved azapirone process provides additional safety and handling advantages.

In the improved process, for example, when the reaction phase has ended, the reaction liquid medium (n-butyl acetate is preferred) is simply washed with water and the inorganic by-products are conveniently and efficiently removed. Following the water wash, the azapirone is extracted with a dilute acid solution, preferably dilute HCI, and the azapirone base is isolated in high yield and purity following basification of the acid extract.

The environmental concerns for the prior process have been reduced in the improved process by the replacement of xylene with, e.g., n-butyl acetate. The use of an imidate salt should present no more environmental concern than the use of imide in the previous process. While care must still be taken in the treatment and disposal of spent aqueous and organic streams in the new process, the environmental impact is much reduced over the prior art processes.

### Description of Specific Embodiments

The process of this invention is illustrated in greater detail by the following examples directed to preferred embodiments of the process steps generally described hereinabove. These examples, however, should not be construed as limiting the scope of the present invention in any way.

### Example 1

Potassium 8-azaspiro[4.5]decane-7,9-dione; (III)

8-Azaspiro[4.5]decane-7,9-dione (167.3 g, 1.0 mole) and toluene (300 mL) were combined in a 1 L flask made inert with nitrogen. To this was added, in ~10 g portions and with vigorous stirring, potassium t-butoxide (116.9 g, 0.99 mole) over a period of 25-30 minutes. The temperature of the mixture increased to ~65°C during the addition. The mixture was then cooled to 25°C and held at this temperature for one hour. The product was filtered and washed with toluene (100 mL). After drying under vacuum at 50-55°C, -200 g of product were obtained as an off-white powdery solid. Yield = 96-99%.

### Example 2

8-[4-[4-(2-Pyrimidinyl)-1-piperazinyl]butyl]-8-azaspiro[4.5]decane-7,9-dione; (Buspirone) 8-(2-Pyrimidinyl)-8-aza-5-azoniaspiro[4.5]decane bromide (II, 48.6 g, .16 moles), potassium 8-azas- piro[4.5]decane-7,9-dione (III, 35.7 g, .174 moles), and butyl acetate (205 mL) were combined and heated to 90-95°C. To insure dryness, vacuum was applied and -25 mL of distillate were collected, then the vacuum was released to nitrogen. The temperature was then increased to reflux (125-126°C) and the mixture was stirred at this temperature for 6-8 hours.

Once the complete reaction was verified by chromatographic analysis¹ , the temperature was reduced to 85-90°C. Water (210 mL) was added, thus lowering the temperature to -55°C. The phases were separated and the lower fraction was back-extracted with butyl acetate (15 mL).

The resulting aqueous was discarded and the two upper phases were combined and extracted with dilute HCI (formed by combining 27.9 mL concentrated HCI and 99.0 mL water). After separating, the upper phase was extracted again with dilute HCI (formed by combining 2.79 mL concentracted HCI and 9.9 mL water). The two rich aqueous fractions were combined.

The product was then precipitated by careful adjustment of the pH to 9.5-9.7 with 30% NaOH solution (total required = -33 mL). The resulting thick yellow slurry was cooled to 0-5°C and filtered. The product was then washed with cold (0-5°C) water (60 mL). The cake was dried to a constant weight at 60-65°C under vacuum. Yield = 59.9 g (95.7%) of buspirone base.

### Example 3

Potassium 3,3-Dimethylglutarimide; (III) 3,3-Dimethylglutarimide (160.0 g, 1.134 moles) and butyl acetate 160 mL) were combined at 25°C. Potassium t-butoxide (132.6 g (95% grade), 1.12 moles) was added, with vigorous agitation, in -10 g portions over 25-30 minutes. During the addition, the temperature of the mixture increased to 65°C. Following addition, the temperature was lowered to 25°C with cooling.

The product was then isolated by filtration and washed with butyl acetate (100 mL). After drying to a constant weight at 50-55°C under vacuum, 149.3 g of an off-white, powdery solid were obtained. Yield = 74.4%.

### Example 4

4,4-Dimethyl-1-[4-[4-(2-Pyrimidinyl)-1-piperazinyl]butyl]-2,6-piperidinedione; (Gepirone) 8-(2-Pyrimidinyl)-8-aza-5-azoniaspiro[4.5]decane bromide (II, 1.59 Kg, 5.31 moles), potassium 3,3-dimethylglutarimide (III, 1.0 Kg, 5.58 moles), and butyl acetate (6.54 L) were combined and heated to reflux (124-126°C). To insure dryness, vacuum was applied and -650 mL of distillate were collected, then the vacuum was released to nitrogen. The mixture was then stirred at 124-126°C for 5-7 hours.

Once the complete reaction was verified by HPLC analysis² , the temperature was reduced to 85-90°C. Water (6.86 L) was added, thus lowering the temperature to 50-55°C. The phases were separated and the lower fraction was back-extracted with butyl acetate (650 mL).

The resulting aqueous was discarded and the two upper phases were combined and extracted with dilute HCI (formed by combining 0.88 L concentrated HCI and 4.7 L water). After separating, the upper phase was extracted again with dilute HCI (formed by combining .09 L concentrated HCI and 0.47 L water). The two rich aqueous fractions were combined.

¹E.g.3" MKGF silica, 9 CH₂CI₂: 1 MeOH, u.v.

²Waters C18ₘ-Bondapak 3.9 mm x 30 cm, 1.5 mL/min of 25% (vol) MeCN: 75% (vol) σ .05 M KH₂P0₄ at pH 5.4.

The product was then precipitated by careful adjustment of the pH to 9.0-9.5 with 30% NaOH solution (total required = -1.14 L). The resulting thick yellow slurry was cooled to 0-5°C and filtered. The product was then washed with cold (0-5°C) water (0.82 L). The cake was dried to a constant weight at 60-65°C under vacuum. Yield = 1.75 Kg (92%) of gepirone base.

## Claims

1. An improved process for large-scale production of azapirones of Formula (I) 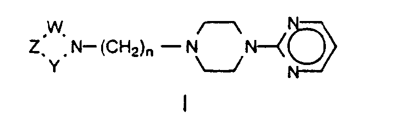 wherein
W and Y are independently selected from carbonyl and sulfonyl;
Z is selected from the group of moieties consisting of 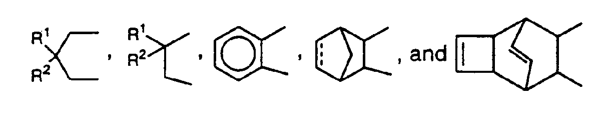 wherein the solid and dotted lines signify either a double or a single covalent bond;
n is the integer 4 or 5; and
R¹ and R² are independently selected from lower alkyl or are taken together as a butyl or pentyl bridge;
the process comprising the anhydrous reaction of a pre-formed potassium salt of formula (III) 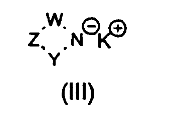 with a spiroquatemary piperazinium salt of Formula (II) 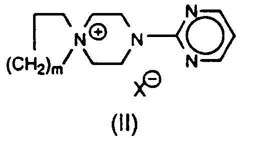 wherein
m is the integer 1 or 2, and
X is a leaving group utilized in organic synthesis;
in an inert reaction medium.

2. The process of claim 1 wherein Z is 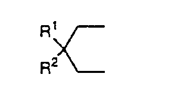 and W and Y are carbonyl.

3. The process of claim 2 wherein R¹ and R² are methyl.

4. The process of claim 2 wherein R¹ and R² are taken together as a butanediyl chain.

5. The process of claim 1, wherein the formula (II) compound is 8-(2-pyrimidinyl)-8-aza-5-azonias- piro[4.5]decane bromide.

6. The process of claim 1, wherein the inert reaction medium is n-butyl acetate.

7. The process of claim 3 wherein the formula (II) compound is 8-(2-pyrimidinyl)-8-aza-5-azoniaspiro[4.5]decane bromide.

8. The process of claim 4 wherein the formula (II) compound is 8-(2-pyrimidinyl)-8-aza-5-azoniaspiro[4.5]decane bromide.

9. The process of claim 7 wherein the inert reaction medium is n-butyl acetate.

10. The process of claim 8 wherein the inert reaction medium is n-butyl acetate.
